# EUROPÄISCHE PATENTSCHRIFT

(11) **EP 2 854 667 B1**
(45) Veröffentlichungstag und Bekanntmachung des Hinweises auf die Patenterteilung: **25.10.2023**
(21) Anmeldenummer: 13726760.5
(22) Anmeldetag: 29.05.2013
(51) Int. Cl.: A61B 17/34, A61B 18/26, A61B 1/00, A61B 90/00

(54) **PUNKTIONSNADELSYSTEM**
PUNCTURE NEEDLE SYSTEM
SYSTÈME D'AIGUILLE DE PONCTION

(30) Priorität: 29.05.2012 DE 102012104621
(43) Veröffentlichungstag der Anmeldung: 08.04.2015
(73) Patentinhaber: Polydiagnost Entwicklungs-, Produktions-, Vertriebs-, und Servicegesellschaft für Medizinelektronische Diagnostik- und, 85276 Pfafenhofen (DE)
(72) Erfinder: SCHAAF, Hansgeorg, 85293 Reichertshausen (DE)
(74) Vertreter: Winter, Brandl - Partnerschaft mbB
(86) Internationale Anmeldenummer: PCT/EP2013/061116
(87) Internationale Veröffentlichungsnummer: WO 2013/178704

(56) Entgegenhaltungen:
- EP-A1- 2 147 654
- WO-A1-2009/024107
- WO-A2-2008/002681
- DE-A1-102009 060 921
- DE-U1- 9 112 976
- US-A1- 2004 204 629
- MAHESH R DESAI ET AL: "Single-Step Percutaneous Nephrolithotomy (Microperc): The Initial Clinical Report", JOURNAL OF UROLOGY, Bd. 186, Nr. 1, Juli 2011 (2011-07), Seiten 140-145, XP028378669, ISSN: 0022-5347, DOI: 10.1016/J.JURO.2011.03.029 [gefunden am 2011-03-10]

## Beschreibung

Die Erfindung betrifft ein Punktionsnadelsystem zur Punktion eines Organs, insbesondere einer Niere, gemäß dem Oberbegriff des Anspruchs 1.

Nach epidemiologischen Studien steigt die Zahl von Patienten mit Harnstein- /Nierensteinerkrankungen stetig an. Diese Zunahme wird mit veränderten Ernährungs- und Lebensgewohnheiten erklärt. Obwohl viele Harn-/Nierensteine den Körper auf natürliche Weise ohne Eingriff passieren können, ist in einer Vielzahl von Fällen zur Vermeidung von Komplikationen ein Eingriff zur Entfernung/Zerkleinerung der Harnsteine erforderlich.

Zur nichtinvasiven Zerkleinerung von Nierensteinen wird die extrakorporale Stoßwellen-Lithotripsie verwendet, die in den 80er Jahren entwickelt wurde. Dabei werden über einen Lithotripter außerhalb des Körpers Druckwellen erzeugt, die in Richtung auf den Nierenstein fokussiert sind und diesen in eine Vielzahl kleinere Partikel fragmentieren. Diese werden dann auf natürliche Weise aus dem Harntrakt ausgeschieden.

Nachteil dieses Verfahrens ist, dass es beim Abgang dieser Steinfragmente zu Koliken kommen kann und dass eine vollständige Entfernung der Steinfragmente nicht gewährleistet ist.

Insbesondere in Fällen, in denen sich der Nierenstein in einer für eine extrakorporale Stoßwellen-Lithotripsie nicht zugänglichen Lage befindet oder wenn er zu groß für eine derartige Behandlung ist, findet die so genannte perkutane Nephrolithotomie (PCNL) Anwendung. Dabei wird über eine Punktionsnadel ein Tunnel direkt zur Niere ausgebildet und durch diesen Tunnel ein Nephroskop eingesetzt, so dass der Stein direkt lokalisiert und entfernt wird. Dafür können starre und flexible Endoskope eingesetzt werden. Als besonders geeignet hat sich das von der Anmelderin unter der Marke "Polyscope^{®}" vertriebene flexible Endoskop erwiesen.

In dem Fall, in dem der Nierenstein zu groß für eine direkte Entnahme mittels eines Zängchens oder Körbchens ist, muss dieser zunächst fragmentiert werden. Dies kann durch Laser-, Ultraschall-, pneumatische oder elektrohydraulische Lithotripsie erfolgen. Einzelheiten zu diesen Verfahren sind dem Aufsatz "Epidemiologische, instrumentelle Therapie und Metaphylaxe des Harnsteinleidens"; Stephan C. Müller; Deutsches Ärzteblatt, Jg. 101, Heft 19, 7. Mai 2004 entnehmbar.

Unter dem Produktnamen "Lithoclast^{®}" wird ein pneumatischer Lithotripter angeboten, der bei sehr geringem Außendurchmesser (3 Fr) eine effektive Zerkleinerung ermöglicht. Bei der Weiterentwicklung LITHOCLAST^{®}MASTER werden drei elementare Lithotripsiefunktionen, d.h. ein pneumatischer Lithotripter der vorbeschriebenen Bauweise, ein Ultraschall-Lithotripter und ein Absaugsystem kombiniert, um die Nierensteine bzw. Nierensteinfragmente entfernen zu können.

In dem Fall, in dem die Steine im mittleren und unteren Ureter angeordnet sind, ist unter Umständen keine Punktion erforderlich, da dann ein Ureteroskop durch die Harnröhre hindurch in den Ureter eingeführt werden kann. Diese Ureteroskopie hat jedoch den Nachteil, dass nur vergleichsweise kleine Steine über Körbchen oder Zängchen direkt entnommen werden können und bei größeren Steinen wiederum eine Lithotripsie erforderlich ist, wobei die Steinfragmente dann ausgespült werden müssen und im Ureter hängen bleiben können - bei größeren Steinen und/oder ungünstigen Lagen der Steine im Harntrakt wird daher weiterhin die minimalinvasive PCNL bevorzugt.

Der wichtigste Schritt zur Vermeidung von Komplikationen bei der PCNL ist dabei die anatomisch korrekte Ausbildung des Kanals zu dem Nierenstein über die Punktionsnadel. Bei der herkömmlichen PCNL wird die korrekte Position der Punktionsnadel mit Bezug dem zu entfernenden Nierenstein über Ortungshilfsmittel, wie beispielsweise Röntgenkontrolle, Fluoroskopie (Kontrastmittel) oder Ultraschall kontrolliert - die Qualität des Zugangs kann jedoch erst dann überprüft werden, wenn der mit den herkömmlichen Techniken erstellte Kanal bougiert ist und das Nephroskop eingesetzt ist. Nach der Ausbildung dieses bougierten Zugangs ist allerdings eine nachträgliche Korrektur nicht oder nur mit erheblichem Aufwand möglich.

Unter dem Namen "Microperc^{®}" wird von der Anmelderin ein Punktionsnadelsystem angeboten, bei dem eine Punktionsnadel, im Folgenden Stilett genannt, in einen Schaft eingesetzt wird, wobei das distale Ende aus dem Schaft auskragt. Der Schaft und das Stilett sind jeweils mit Luerlock-Kupplungselementen ausgeführt, so dass Schaft und Stilett koaxial verbunden werden können. Die Optik kann dann an einem axialen proximalen Endabschnitt der Luerlock-Kupplung des Stiletts angesetzt werden, wobei in bekannter Weise die eigentliche Optik über einen Shifter in der Länge so eingestellt wird, dass die Punktion optisch überwacht werden kann. D.h. die Optik endet im Bereich des proximalen Endabschnitts des Stiletts. Nach der Punktion wird das Stilett mit der damit verbundenen Optik aus dem Schaft herausgezogen und an dessen Luerlock-Kupplungselement ein Mehrwegeadapter angesetzt. Die Optik wird dann wieder durch einen axialen Anschluss des Mehrwegeadapters in den Schaft eingeführt und mit dem Adapter gekuppelt. Problematisch dabei ist, dass die Länge der Optik beim Punktieren so eingestellt ist, dass sie beim Einsetzen an den Mehrwegeadapter aus dem distalen Ende des Schaftes herausragt. Da der Schaft, wie vorstehend erläutert, in der Nähe des Nierensteins angeordnet ist, kann es durch die dann herausgeschobene Optik zu Verletzungen des Patienten kommen. Aus diesem Grund ist es erforderlich, die Länge der Optik gegenüber der Wirklänge in der ersten Konstellation zu verringern - zu dieser Verringerung ist jedoch eine erhebliche Erfahrung erforderlich. Hierzu gibt es auch einen klinischen Bericht von Mahesh E. Desai et al. mit dem Titel "Single-Step Percutaneous Nephrolithotomy (Microperc): The Initial Clinical Report", erschienen im Journal of Urology im Bd. 186, Nr. 1, Seiten 140-145, datiert auf Juli 2011.

Weiterer gattungsgemäßer Stand der Technik ist durch die WO 2009/024107 A1 und die DE 10 2009 060921 A1 bekannt.

Demgegenüber liegt der Erfindung die Aufgabe zugrunde, ein Punktionsnadelsystem zu schaffen, mit dem eine Verletzungsgefahr für den Patienten verringert ist.

Diese Aufgabe wird durch ein Punktionsnadelsystem mit den Merkmalen des Patentanspruches 1 gelöst.

Erfindungsgemäß hat das Punktionsnadelsystem einen Schaft, in den in einer ersten Konstellation einen Punktionsnadel (Stilett) eingeführt ist, die ihrerseits eine Optik aufnimmt. Das System hat des Weiteren einen Mehrwegeadapter, der in einer zweiten Konstellation an den Schaft angesetzt ist und an den dann die Optik wiederum angesetzt werden kann. Dieser Mehrwegeadapter hat einen Spülanschluss und einen Anschluss für eine Einrichtung zum Desintegrieren, beispielsweise des Nierensteins. Erfindungsgemäß sind die Bauelemente derart aufeinander abgestimmt, dass dann, wenn die Optik mit der in der ersten Konstellation eingestellten Wirklänge an den Mehrwegeadapter angesetzt wird (zweite Konstellation) deren Wirklänge gleich oder kleiner ist als diejenige des Schaftes, so dass die Optik mit dem Schaftende abschließt oder gegenüber diesen in den Schaft hinein verschoben ist.

Dadurch ist sichergestellt, dass die Optik allenfalls durch aktives Verschieben in Richtung distales Ende des Schaftes verschoben werden kann, eine versehentliche Verletzung des Patienten durch Ansetzen der Optik ist aber ausgeschlossen, da deren Wirklänge kürzer als diejenige des Schaftes ist.

Zusätzlich lässt sich die Sicherheit für den Patienten dadurch weiter erhöhen, dass der Mehrwegeadapter als Y-Adapter mit Axialanschluss ausgeführt ist, der an das Kupplungselement des Schaftes ansetzbar ist und, dass das Punktionsnadelsystem mit einem Schutzrohr für eine Laserfibre oder eine andere Einrichtung zum thermischen Desintegrieren ausgeführt ist, die in der zweiten Konstellation an einen der Y-Anschlüsse des Mehrwegeadapters ansetzbar ist. Dabei steht das Schutzrohr über die Optik hinaus, so dass diese nicht durch die Laserfibre thermisch beschädigt werden kann.

Bei einem Ausführungsbeispiel der Erfindung ist das Stilett mit einem T-Adapter ausgeführt, der mit einem entsprechenden Kupplungselement des Schaftes verbindbar ist, wobei das distale Ende des Stiletts im angesetzten Zustand aus dem Schaft auskragt.

In der zweiten Konstellation ist die Optik an einen axialen Anschluss des Mehrwegeadapters angesetzt.

Auch ist es von Vorteil, wenn das Punktionsnadelsystem auch mit einer Laserfibre oder dergleichen ausgeführt ist, die in das Schutzrohr einführbar ist. Dazu ist letzteres mit einer Kupplung ausgeführt, die mit einer entsprechenden Kupplung der Laserfibre oder dergleichen verbindbar ist.

In dem Fall, in dem eine thermische Desintegration nicht ausreicht, kann das Punktionsnadelsystem zusätzlich mit einem größeren Schaft und einem Dilatator ausgeführt sein; an den Schaft können dann andere mechanische Desintegrierer oder dergleichen angesetzt werden.

Ein bevorzugtes Ausführungsbeispiel der Erfindung wird im Folgenden anhand schematischer Zeichnungen näher erläutert. Es zeigen:
Figur 1 einen Schaft und ein Stilett eines erfindungsgemäßen Punktionsnadelsystems, links ist ein Schaft mit einer Luerlock-Kupplung dargestellt, rechts ist das Stilett mit einer entsprechenden Luerlock-Kupplung und einem T-Adapter gezeigt;
Figur 2 den Schaft aus Figur 1 mit eingesetztem Stilett, wobei das distale Ende des Stiletts über dasjenige des Schaftes hinaussteht;
Figur 3 das erfindungsgemäße Punktionsnadelsystem in einer ersten Konstellation, in der eine Optik an einen axialen Anschluss des T-Adapters angesetzt ist, wobei die Axiallänge über einen Shifter so einstellbar ist, dass das distale Ende der Optik kurz vor dem distalen Ende des Stiletts endet und somit nicht auskragt;
Figur 4 das Punktionsnadelsystem in einer Zwischenposition, nachdem das Stilett und die Optik aus dem Schaft herausgezogen sind und an letzterem ein Mehrwegeadapter angesetzt ist, der als Y-Adapter mit zwei Y-Anschlüssen und einem axialen Anschluss ausgeführt ist;
Figur 5 eine zweite Konstellation, gemäß der die Optik mit der gegenüber der ersten Konstellation unveränderten Länge an den Mehrwegeadapter angesetzt ist; in der Mitte links ist der distale Endabschnitt des Punktionsnadelsystems gezeigt, demgemäß kragt die Optik in der zweiten Konstellation nicht - wie beim Stand der Technik - aus dem distalen Ende des Schaftes aus sondern schließt bündig mit diesem ab oder ist gegenüber diesem zurückgezogen;
Figur 6 das Punktionsnadelsystem aus Figur 5 mit einem zusätzlichen, noch nicht angesetzten Schutzrohr;
Figur 7 das Punktionsnadelsystem, wobei das Schutzrohr des Mehrwegeadapters angesetzt ist, dabei ist dieses Schutzrohr über eine geeignete Kupplung an einen der Y-Anschlüsse des Mehrwegeadapters angesetzt und durchsetzt den Schaft;
Figur 8 den distalen Endabschnitt des Schaftes mit auskragendem Schutzrohr;
Figur 9 eine Laserfibre, die in das Schutzrohr teilweise eingeführt ist, über die eine thermische Desintegration des Nierensteins erfolgt; diese Laserfibre und das Schutzrohr sind mit geeigneten Kupplungselementen, beispielsweise einem Luerlock ausgeführt;
Figur 10 zeigt den gekoppelten Zustand, in dem die Laserfibre etwas aus dem Schutzrohr herausragt; durch das Schutzrohr ist eine thermische Beschädigung der Optik, die ebenfalls im Mündungsbereich des Schaftes angeordnet ist, ausgeschlossen, so dass die Desintegration ohne Beschädigung der Optik erfolgt und über die Optik eine exakte Kontrolle ermöglicht ist;
Figur 11 einen 8-Fr-Satz mit Dilatator und größerem Schaft sowie einem entsprechenden Mehrwegadapter und
Figur 12 das Punktionsnadelsystem, bei dem an den dickeren Schaft an einen axialen Anschluss des Mehrwegeadapters ein mechanischer Desintegrierer angeordnet ist; in diesem Fall wird die Optik in einen der Y-Anschlüsse des Mehrwegeadapters eingeschoben.

Das erfindungsgemäße Punktionsnadelsystem 1 wird im Folgenden anhand der einzelnen Bauelemente erläutert, aus denen dieses System zusammengesetzt ist.

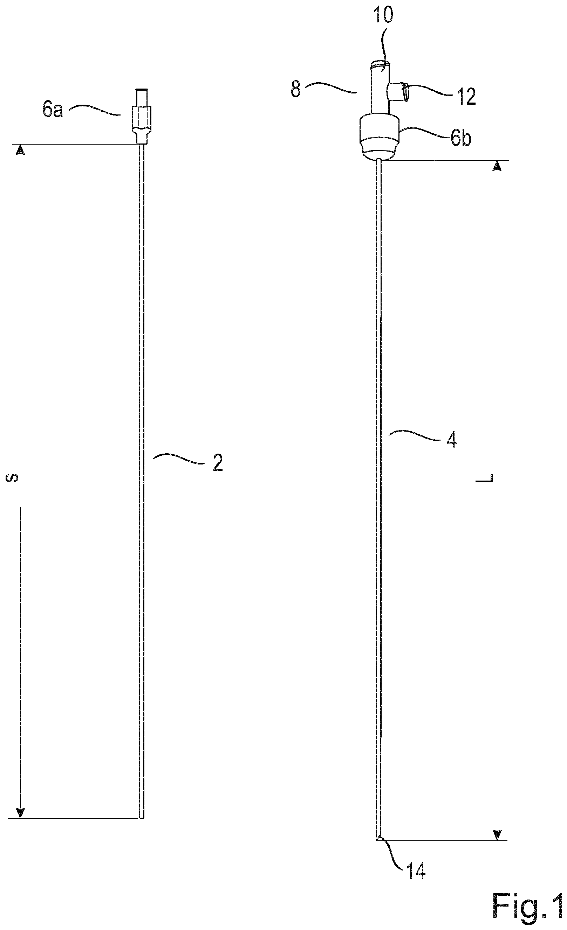

Gemäß Figur 1 weist das Punktionsnadelsystem 1 einen Schaft 2 und ein Stilett 4 auf. Der Innendurchmesser des hohlen Schaftes 2 ist so ausgelegt, dass das Stilett 4 einsetzbar ist. Am proximalen Endabschnitt des Schaftes 2 ist eine herkömmliche Luer-Kupplung 6 oder besser gesagt, ein Teil einer derartigen Kupplung dargestellt. Das Stilett 4 trägt am proximalen Endabschnitt einen T-Adapter 8, der einen axialen Anschluss 10 und einen radialen Anschluss 12 aufweist. Der T-Adapter 8 ist mit einem Luer-Kupplungselement 6b ausgeführt, das zur Luer-Kupplung 6a des Schaftes 2 komplementär ist. Auch das Stilett 4 ist hohl ausgeführt und hat eine in der Darstellung gemäß Figur 1 schräg angestellte Spitze 14. Die Axiallänge L des Stilettes ist größer als diejenige des Schaftes 2 ausgeführt, so dass beim Einsetzen des Stiletts 4 in den Schaft 2 die Spitze 14 aus dem Mündungsbereich des Schaftes 2 hervorsteht. In dieser Darstellung ist auch erkennbar, dass das Stilett 4 hohl ausgeführt ist.

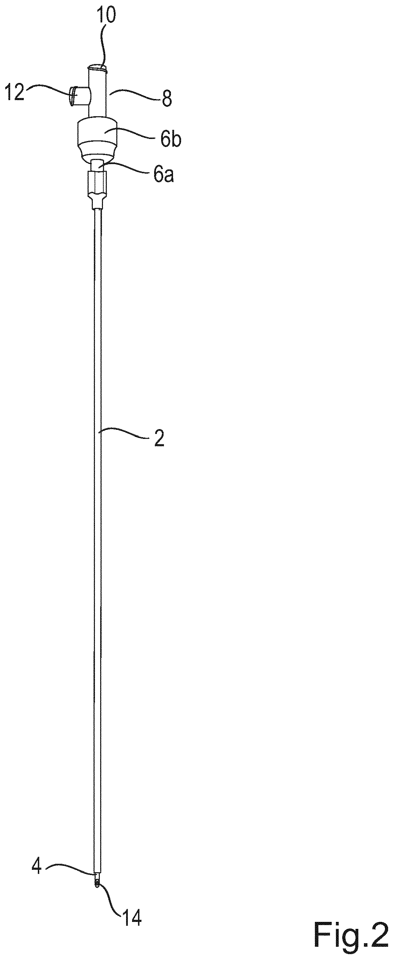

In der in Figur 2 dargestellten zusammen gesetzten Position stehen die Luer-Kupplungselemente 6a, 6b in Wirkeingriff.

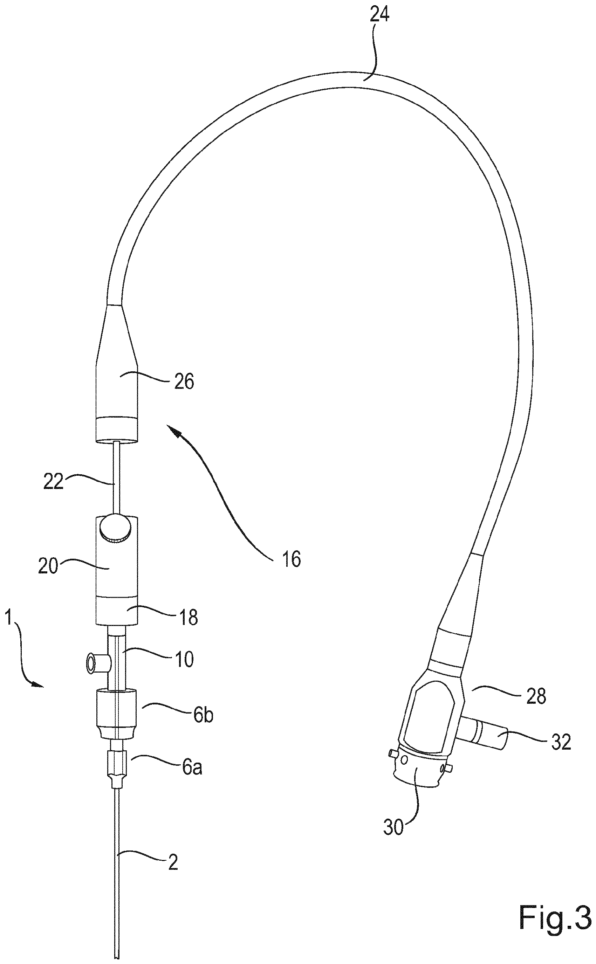

Figur 3 zeigt das Punktionsnadelsystem gemäß Figur 2, wobei eine Optik 16 eingesetzt ist. Der prinzipielle Aufbau der Optik 16 ist bereits aus den Polyscope^{®}-System der Anmelderin bekannt, so dass im Hinblick auf Details dieser Optik auf diesen Stand der Technik und die DE 20 2007 011 781 U1 verwiesen werden kann. Eine derartige Optik 16 hat ein hochflexibles Bündel geordneter Bild- und Lichtfasern, über die der zu untersuchende Bereich beleuchtet und Bildinformationen einem Okular- oder einem angeschlossenen Bildaufzeichnungsgerät zugeleitet werden. Die Befestigung der Optik 16 an dem axialen Anschluss 10 des T-Adapters erfolgt über einen entsprechenden Adapter 18 eines Shifters 20, über den die Wirklänge der Optik einstellbar ist. Das heißt, durch Verstellen des Shifters 20 kann ein aus diesem herausragendes Führungsrohr 22 in Pfeilrichtung verstellt werden, um den distalen Endabschnitt der Optik mit Bezug zur Mündung der Spitze 14 auszurichten. Diese Positionierung kann über die Bildauswertung überwacht werden. Sobald diese SollPosition erreicht ist, wird die Klemmschraube des Shifters 20 mit dem Führungsrohr 22 verspannt, so dass die Optik lagepositioniert ist. Die eigentlichen Bild- und Lichtfasern durchsetzen das Führungsrohr 22 und einen Optikschutzschlauch 24, der über eine Knickschutzhülse 26 mit dem Führungsrohr 22 verbunden ist. An dem vom Shifter 20 entfernten Endabschnitt des Optikschutzschlauches 24 ist dann ein Optikgrundkörper 28 mit einem Okularanschluss 30 und einem Beleuchtungsanschluss 32 angeordnet.

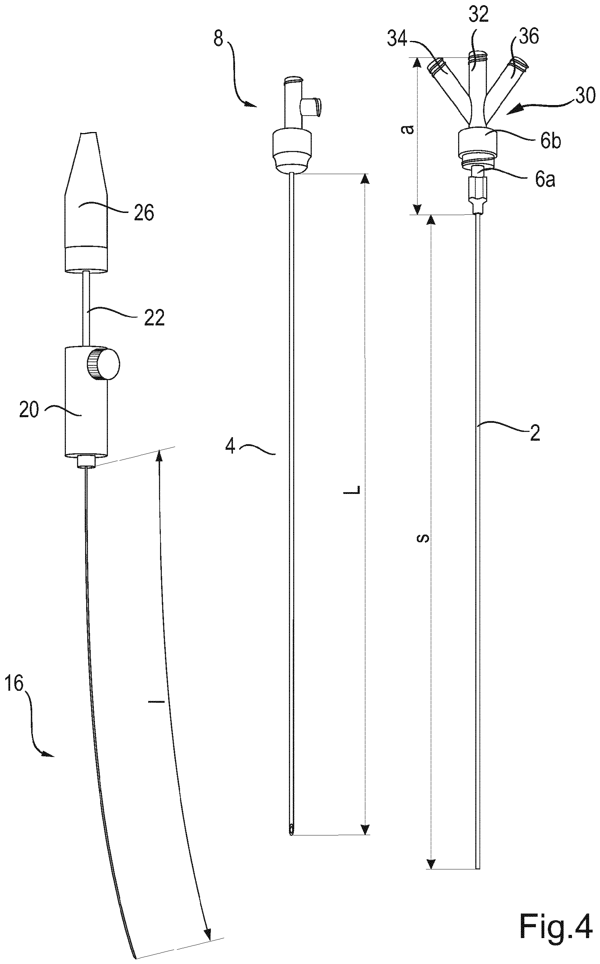

Beim konkreten Ausführungsbeispiel ist das distale Ende der Optik so eingestellt, dass es gerade nicht aus dem Stilett 4 auskragt. Es stellt sich dann eine Wirklänge I der Optik ein, die in Figur 4 gekennzeichnet ist. Wie gesagt, wird diese Wirklänge I mittels des Shifters 20 eingestellt und fixiert. Nach dem Punktieren werden die Optik 16 und das Stilett 4 aus dem Schaft 2 heraus gezogen, wobei auch die Optik 16 aus dem Stilett 4 gezogen wird. Dies erfolgt jeweils durch Lösen der Luer-Kupplungselemente. Der T-Adapter 8 verbleibt gemäß Figur 4 an dem Stilett 4. An dem Schaft 2, der noch im Tunnel zur Niere steckt, wird nun ein Mehrwege-Adapter 30 angesetzt, wobei dieser ebenfalls mit einem Luer-Kupplungselement 6b ausgeführt ist, das mit dem entsprechenden Kupplungselement 6a des Schaftes 2 in Wirkeingriff bringbar ist. Dieser Mehrwege-Adapter 30 hat einen mittigen Axialanschluss 32 und zwei Y-Anschlüsse 34, 36. In einem folgenden Schritt wird dann die aus dem Stilett 4 herausgezogene Optik 16 über den Axialanschluss 32 eingesetzt und über die Luer-Kupplungselemente verriegelt.

Erfindungsgemäß sind dabei die Länge S des Schaftes und die Wirklänge I der Optik 16 so aufeinander abgestimmt, dass ohne Veränderung der Shifterposition 20 das Bild-/Lichtfaserbündel 39 der Optik 16 nicht aus einer Mündung 38 des Schaftes 2 auskragt. Das heißt, die eigentliche Endoskop-Optik, die beispielsweise ein C-MOS-Chip oder eine Linse sein kann, wird von dem Schaft 2 überdeckt. Dies ist ein wesentlicher Vorteil zu herkömmlichen Lösungen, bei denen es, wie eingangs erläutert, durch eine heraus geschobene Optik zu Verletzungen des Patienten kommen kann. Dies wird durch die erfindungsgemäße Konzeption des Punktionsnadelsystems zuverlässig verhindert.

Bei dem beschriebenen Ausführungsbeispiel sind somit mehrere Abmessungen aufeinander abgestimmt. Zum Einen ist die Axiallänge L des Stiletts 4 etwas größer als die Länge S des Schaftes. Zum Anderen ist die Wirklänge der Optik I im angesetzten Zustand so ausgelegt, dass deren distaler Endabschnitt ohne Nachstellen vom Schaft 2 überdeckt ist.

Das heißt, die Länge S des Schaftes 2 plus die Axiallänge a des MehrwegeAdapters 30 sind im Prinzip größer als die Wirklänge I der Optik 16 ausgelegt. Unter der Wirklänge I der Optik wird dabei etwa der Abstand verstanden, den das distale Ende der Optik 16 von dem Luer-Kupplungselement des Shifters 20 hat.

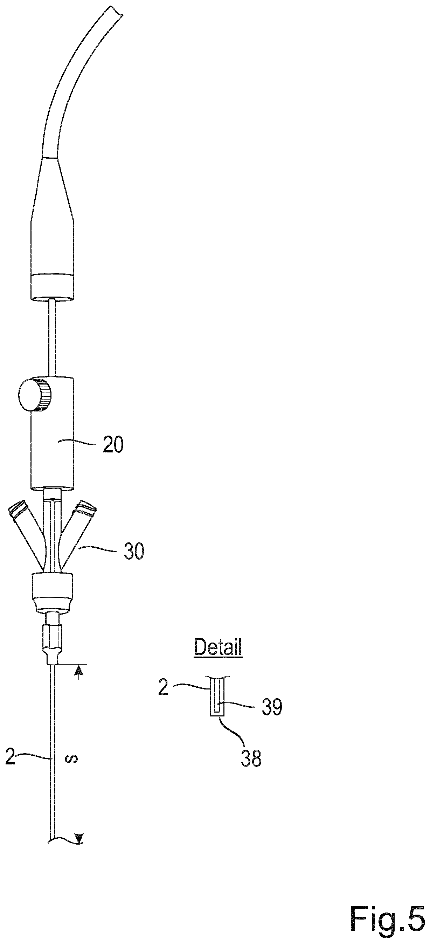

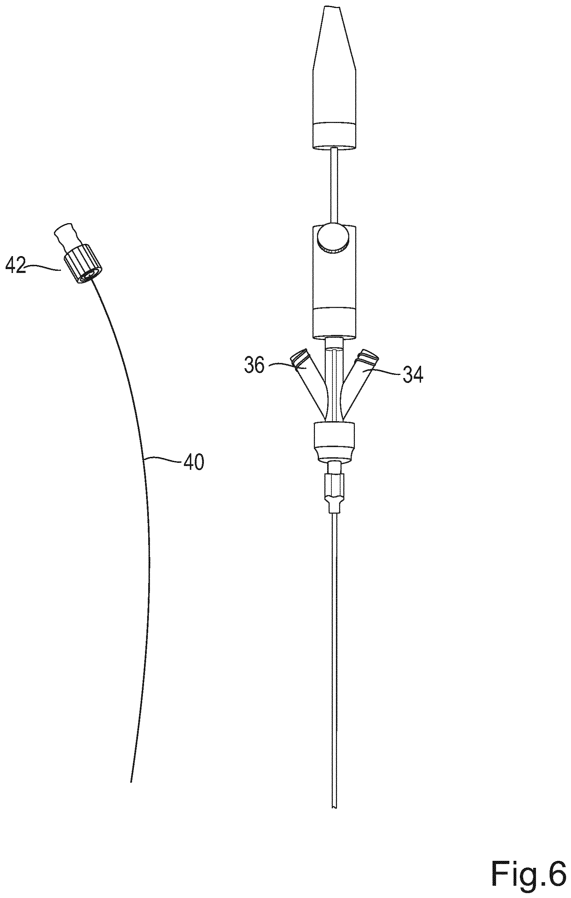

Figur 6 zeigt die Anordnung gemäß Figur 5 mit einem zusätzlichen Schutzrohr 40, das mit einem Kupplungselement 42 ausgeführt ist, das an einem Y-Anschlüsse 34, 36 ansetzbar ist. Diese Kupplungselemente sind dabei nicht als Luer-Kupplung ausgeführt.

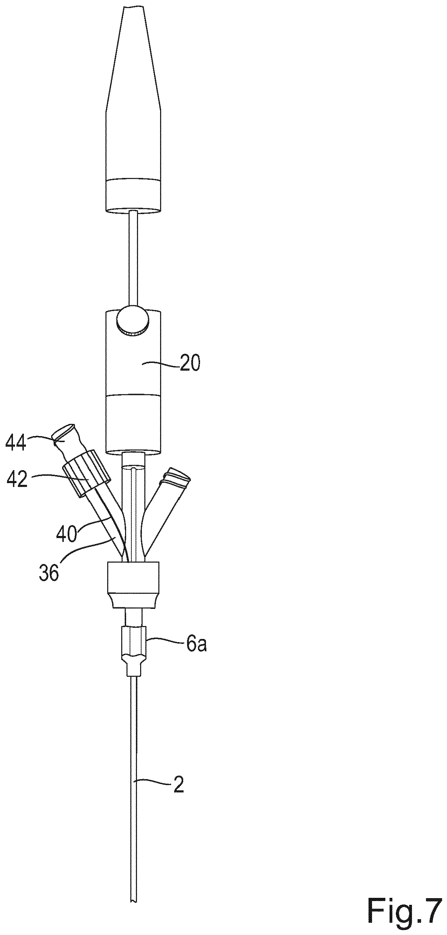

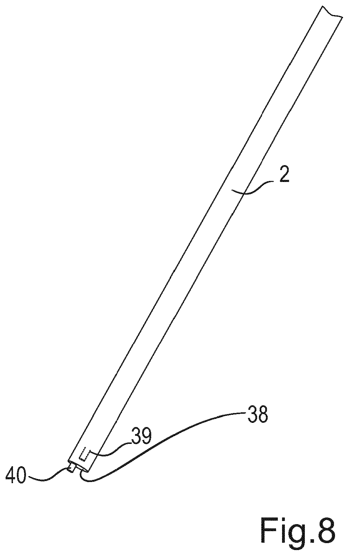

In der Darstellung gemäß Figur 7 ist das Schutzrohr 40 in den Y-Anschluss 36 eingesetzt und über das Kupplungselement 42 verriegelt. Das Kupplungselement 42 hat seinerseits eine weitere geeignete Kupplung 44, an die ein entsprechendes Kupplungselement einer Laserfibre ansetzbar ist, über die die thermische Desintegration des Nierensteins erfolgt. Figur 8 zeigt den distalen Endabschnitt des Schutzrohrs 2. Man erkennt deutlich, dass der Mündungsbereich des Schutzrohrs 40 aus der Mündung 38 des Schaftes 2 auskragt. Die Lage der Optik, genauer gesagt des distalen Endabschnitts des Faserbündels 39 ist gestrichelt angedeutet.

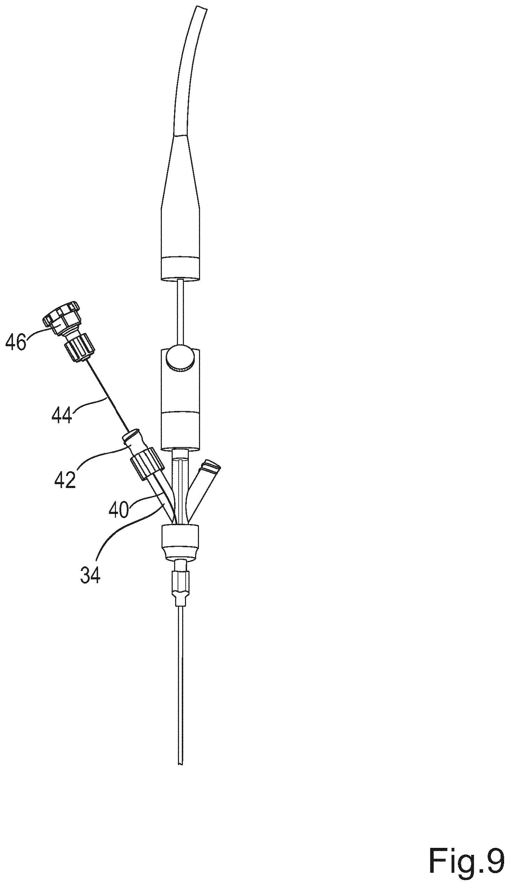

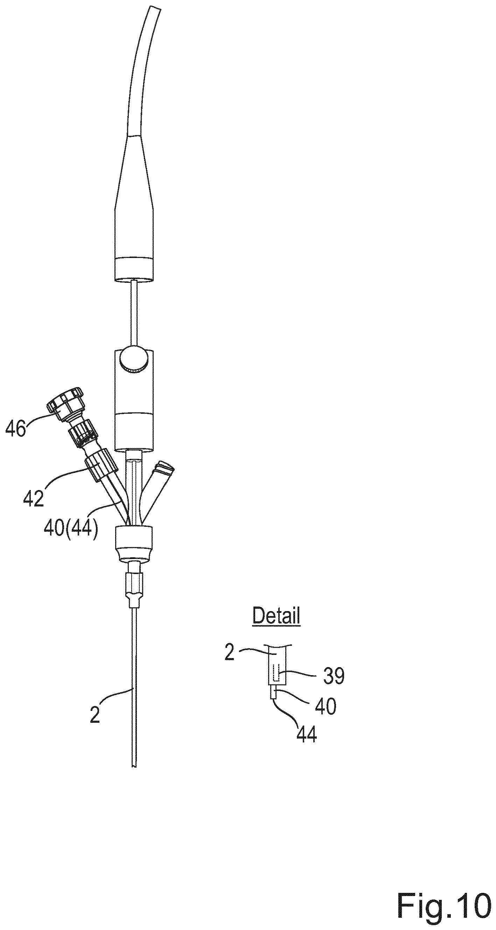

Gemäß Figur 9 wird dann in dieses Schutzrohr 40 die Laserfibre 44 eingesetzt und über die Kupplung 46 mit dem Kupplungslement 42 des Y-Anschlusses 34 verbunden. Der zusammen gefügte Zustand ist in Figur 10 dargestellt. Das Detail zeigt dabei den distalen Endabschnitt des Schaftes 2 etwas vergrößert. Man sieht das auskragende Schutzrohr 40 und die in Axialrichtung etwas daraus hervorstehende Laserfibre 44. Durch dieses Schutzrohr 40 ist gewährleistet, dass während des thermischen Desintegration die Optik nicht beschädigt wird, wobei der Wirkbereich der Laserfibre 44 über die Optik (gestrichelt angedeutet in Figur 10) exakt kontrollierbar ist.

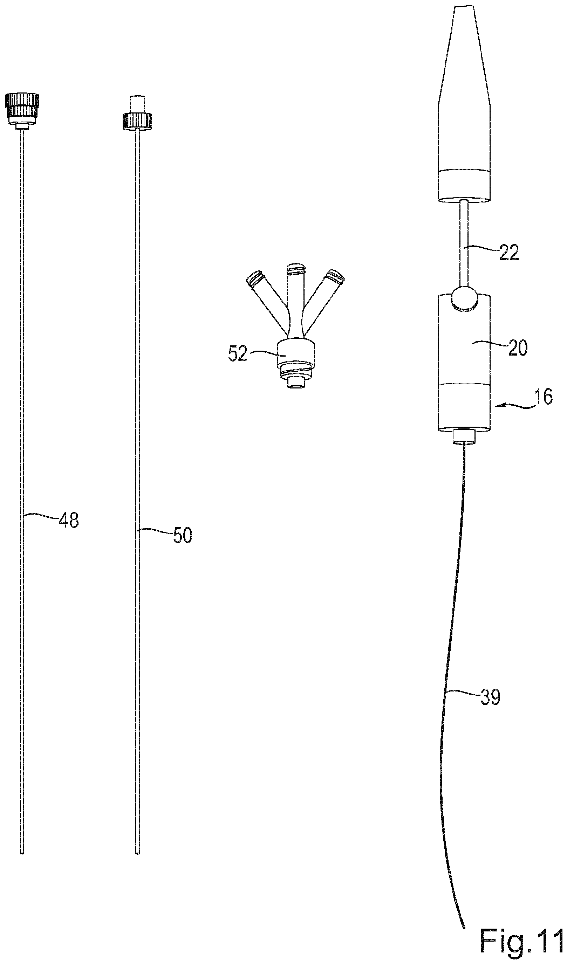

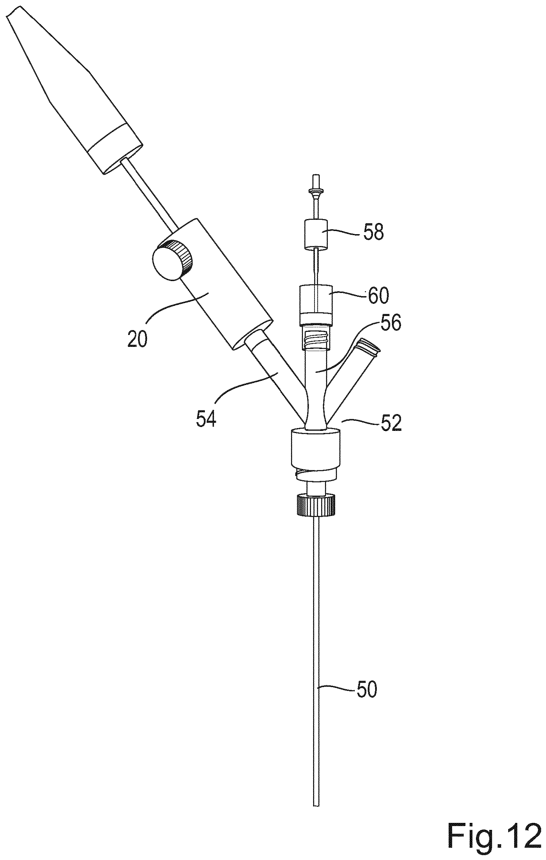

Figur 11 zeigt weiteres Zubehör des erfindungsgemäßen Punktionsnadelsystems 1 mit einem 8-Fr-Satz bestehend aus einem Dilatator 48, einem entsprechenden Schaft 50 sowie einem geeigneten Mehrwege-Adapter 52. Diese Elemente sind wiederum mit geeigneten Kupplungselementen, beispielsweise Luer-Kupplungselementen oder dergleichen ausgeführt, um sie miteinander verbinden zu können. Auch zu diesem 8-Fr-Satz gehört die Optik 16, die in der vorbeschriebenen Weise ausgebildet ist. Figur 12 zeigt den 8-Fr-Satz mit dem dickeren Schaft 50, an den der "dickere" Mehrwege-Adapter 52 angesetzt ist. Dieses Mal ist die Optik mit dem Shifter 20 an einen der Y-Anschlüsse 54 angesetzt, wobei die Relativpositionierung des distalen Endes der Optik mit Bezug zur Mündung des Schaftes 50 in der vorbeschriebenen Weise erfolgt. Auch hier ist die Wirklänge der Optik I vorab so gewählt, dass diese beim Einsetzen nicht aus dem gesetzten Schaft 50 auskragt. An einen axialen Anschluss 56 des MehrwegeAdapters ist ein mechanischer Zerstörer (Desintegrierer) 58 über eine geeignete Kupplung 60 angesetzt, über den die Nierensteine desintegriert werden können.

Wie gesagt, sind die Axiallängen der verwendeten Schäfte mit Bezug zur Optik, die auf die Wirklänge des Stiletts 4 eingestellt ist, so abgestimmt, dass bei Ansetzen der Optik an den jeweiligen Schaft die Optik nicht auskragt. Das heißt, Stilett 4, Schäfte 2, 50 und die Wirklänge der Optik I sind in der erfindungsgemäßen Weise auf einander abgestimmt. Durch die erfindungsgemäßen Maßnahmen ist gewährleistet, dass eine versehentliche Verletzung des Patienten durch eine Fehleinstellung der Optiklänge ausgeschlossen ist.

Um das Umlenken der Optik und des Schutzrohres innerhalb der beschriebenen Mehrwegeadapter zu erleichtern, können diese im Bereich der inneren Umlenkungen so ausgeführt sein, dass diese Umlenkung "sanft" ohne Knicke erfolgt.

Bei dem dargestellten Ausführungsbeispiel ist der Außendurchmesser des Schaftes 2 kleiner als 2,0 mm, konkret etwa 1,65 mm ausgeführt. Ein derart kompaktes Punktionsnadelsystem 1 mit den beschriebenen Funktionen ist ohne Vorbild.

Offenbart ist ein Punktionsnadelsystem mit einer Optik, einer Punktionsnadel oder dergleichen und einem Schaft, wobei die Wirklängen dieser Elemente so aufeinander abgestimmt sind, dass bei Einsetzen der Optik in den Schaft die Optik nicht über den Mündungsbereich des Schaftes auskragt.

### Bezugszeichen

- 1: Punktionsnadelsystem
- 2: Schaft
- 4: Stilett
- 6: Luer-Kupplung
- 8: T-Adapter
- 10: axialer Anschluss
- 12: radialer Anschluss
- 14: Spitze
- 16: Optik
- 18: Adapter
- 20: Shifter
- 22: Führungsrohr
- 24: Optikschutzschlauch
- 26: Knickschutzhülse
- 28: Optikgrundkörper
- 30: Mehrwege-Adapter
- 32: Axialanschluss
- 34: Y-Anschluss
- 36: Y-Anschluss
- 38: Mündung
- 39: Faserbündel
- 40: Schutzrohr
- 42: Kupplungselement
- 44: Laserfibre
- 46: Kupplung
- 48: Dilatator
- 50: Schaft
- 52: Mehrwege-Adapter
- 54: Y-Anschluss
- 56: Axialanschluss
- 58: Zerstörer
- 60: Kupplung

## Patentansprüche

1. Punktionsnadelsystem (1) mit einem Schaft (2), einer Punktionsnadel (4), einer Optik (16), einem Shifter (20), über den eine Wirklänge (I) der Optik (16) einstellbar ist, und einem Mehrwegeadapter (30), wobei in einer ersten Konstellation die Punktionsnadel (4) in den Schaft (2) eingeführt ist, die ihrerseits die Optik (16) aufnimmt, und wobei der Mehrwegeadapter (30) in einer zweiten Konstellation an den Schaft (2) angesetzt ist und so vorbereitet ist, dass an ihn die Optik (16) angesetzt werden kann, wobei der Mehrwegeadapter (30) ferner einen Anschluss für eine Einrichtung zum Desintegrieren hat und in der zweiten Konstellation die Optik (16) und die Einrichtung zum Desintegrieren den Schaft (2) durchsetzen, und wobei der Mehrwegeadapter (30) als Y-Adapter mit Axialanschluss (32) ausgeführt ist und an ein Kupplungselement des Schaftes (2) anschließbar ist, **dadurch gekennzeichnet, dass** der Schaft (2), die Punktionsnadel (4) und die Optik (16) derart aufeinander abgestimmt sind, dass die Wirklänge (I) der Optik (16) derart mittels des Shifters (20) eingestellt und fixiert ist, dass in der ersten Konstellation ein distales Ende der Optik (16) gerade nicht aus der Punktionsnadel (4) auskragt und die Optik (16) in der zweiten Konstellation, in der die Optik (16) an den Mehrwegeadapter (30) angesetzt ist, mit einem Ende des Schafts (2) abschließt oder gegenüber diesem Ende etwas in den Schaft (2) hinein verschoben ist, und wobei ferner ein Schutzrohr (40) für eine Laserfibre (44) oder eine andere Einrichtung zum thermischen Desintegrieren, die in der zweiten Konstellation an einem Y-Anschluss (36) des Mehrwegeadapters (30) ansetzbar ist, vorgesehen ist, und das Schutzrohr (40) eine derartige Länge aufweist, dass es in der zweiten Konstellation über die Optik (16) hinaussteht.

2. Punktionsnadelsystem (1) nach Patentanspruch 1, wobei die Punktionsnadel (4) mit einem T-Adapter und der Schaft (2) mit einem entsprechenden Kupplungselement zum Koppeln mit dem T-Adapter ausgeführt sind, wobei das distale Ende der Punktionsnadel (4) im angesetzten Zustand aus dem Schaft (2) auskragt.

3. Punktionsnadelsystem (1) nach einem der vorhergehenden Patentansprüche, wobei die Optik (16) in der zweiten Konstellation an den Axialanschluss (32) des Mehrwegeadapters (30) angesetzt ist.

4. Punktionsnadelsystem (1) nach einem der vorhergehenden Patentansprüche, wobei das Schutzrohr (40) in der zweiten Konstellation aus dem Schaft (2) auskragt.

5. Punktionsnadelsystem (1) nach Patentanspruch 4, mit einer Laserfibre (44) zum Einführen in das Schutzrohr (42), das eine Kupplung hat, die mit einer entsprechenden Kupplung der Laserfibre (44) verbindbar ist.

6. Punktionsnadelsystem (1) nach einem der vorhergehenden Patentansprüche, mit einem weiteren Schaft (50) mit größerem Durchmesser als derjenige des erstgenannten Schaftes (2) und einem Dilatator (48).

7. Punktionsnadelsystem (1) nach einem der vorhergehenden Patentansprüche, mit einem mechanischen Desintegrierer.

8. Punktionsnadelsystem (1) nach einem der vorhergehenden Ansprüche, wobei ein Außendurchmesser des Schaftes (2) kleiner als 2.0 mm, vorzugsweise kleiner 1,7 mm, besonders vorzugsweise etwa 1,65 mm ist.

## Claims

1. Puncture needle system (1) including a shaft (2), a puncture needle (4), optics (16), a shifter (20) via which an effective length (I) of the optics (16) can be adjusted, and a multiway adapter (30), wherein in a first constellation the puncture needle (4) is inserted into the shaft (2), which in turn receives the optics (16), and wherein in a second constellation the multiway adapter (30) is attached to the shaft (2) and is prepared so that the optics (16) can be attached to it, wherein the multiway adapter (30) moreover has a connection for a device for disintegration and wherein, in the second constellation, the optics (16) and the device for disintegration pass through the shaft (2), and wherein the multiway adapter (30) is designed as a Y-adapter with an axial connection (32) and can be connected to a coupling element of the shaft (2), **characterized in that** the shaft (2), the puncture needle (4) and the optics (16) are matched to one another such that the effective length (I) of the optics (16) is set and fixed by means of the shifter (20) in such a way that in the first constellation, a distal end of the optics (16) just does not protrude from the puncture needle (4) and, in the second constellation, in which the optics (16) is attached to the multiway adapter (30), closes with one end of the shaft (2) or is displaced somewhat into the shaft (2) with respect to this end, and wherein moreover a protective tube (40) is provided for a laser fiber (44) or another device for thermal disintegration which, in the second constellation, can be attached to a Y-connection (36) of the multi-way adapter (30), and the protective tube (40) has a length such that it protrudes beyond the optics (16) in the second constellation.

2. The puncture needle system (1) according to claim 1, wherein the puncture needle (4) is carried out with a T-adapter and the shaft (2) is carried out with a corresponding coupling element for coupling with the T-adapter, the distal end of the puncture needle (4) protruding from the shaft (2) in the attached state.

3. The puncture needle system (1) according to any of the preceding claims, wherein the optics (16) in the second constellation is attached to the axial connection (32) of the multi-way adapter (30).

4. The puncture needle system (1) according to any of the preceding claims, wherein the protective tube (40) in the second constellation protrudes from the shaft (2).

5. The puncture needle system (1) according to claim 4, comprising a laser fiber (44) for insertion into the protective tube (42), which has a coupling which is connected to the corresponding coupling of the laser fiber (44).

6. The puncture needle system (1) according to any of the preceding claims, comprising a further shaft (50) having a larger diameter than that of the first-mentioned shaft (2) and a dilator (48).

7. The puncture needle system (1) according to any one of the preceding claims, comprising a mechanical disintegrator.

8. The puncture needle system (1) according to any one of the preceding claims, wherein an outer diameter of the shaft (2) is smaller than 2.0 mm, preferably smaller than 1.7 mm, particularly preferably about 1.65 mm.

## Revendications

1. Système d'aiguille de ponction (1) avec une tige (2), une aiguille de ponction (4), une optique (16), un commutateur (20), par l'intermédiaire duquel une longueur effective (I) de l'optique (16) est réglable, et un adaptateur à plusieurs voies (30), dans lequel dans une première constellation l'aiguille de ponction (4) est insérée dans la tige (2), laquelle accueille de son côté l'optique (16), et dans lequel l'adaptateur à plusieurs voies (30) est appliqué dans une deuxième constellation au niveau de la tige (2) et ainsi préparé de sorte qu'au niveau de celle-ci l'optique (16) peut être appliquée, dans lequel l'adaptateur à plusieurs vois (30) présente en outre un raccordement pour un appareil de désintégration et dans la deuxième constellation l'optique (16) et l'appareil de désintégration traversent la tige (2), et dans lequel l'adaptateur à plusieurs voies (30) est réalisé en tant qu'adaptateur en Y avec un raccordement axial (32) et peut être raccordé à un élément de couplage de la tige (2), **caractérisé en ce que** la tige (2), l'aiguille de ponction (4) et l'optique (16) sont coordonnées de telle sorte que la longueur effective (I) de l'optique (16) est ajustée et fixée au moyen du commutateur (20) de telle sorte que dans la première constellation une extrémité distale de l'optique (16) ne dépasse pas de l'aiguille de ponction et l'optique (16) dans la deuxième constellation, dans laquelle l'optique (16) est appliquée au niveau de l'adaptateur à plusieurs voies (30), se termine avec une extrémité de la tige (2) ou par rapport à cette extrémité quelque chose est décalé dans la tige (2), et dans lequel en outre un tuyau de protection (40) pour une fibre laser (44) ou un autre appareil de désintégration thermique, qui peut être appliqué dans la deuxième constellation au niveau d'un raccordement en Y (36) de l'adaptateur à plusieurs voies (30), est prévu, et le tuyau de protection (40) présente une longueur de telle sorte qu'il fait saillie dans la deuxième constellation au-delà de l'optique (16).

2. Système d'aiguille de ponction (1) selon la revendication 1, dans lequel l'aiguille de ponction (4) est réalisée avec un adaptateur en T et la tige (2) est réalisée avec un élément de couplage correspondant pour un couplage avec l'adaptateur en T, dans lequel l'extrémité distale de l'aiguille de ponction (4) fait saillie depuis la tige (2) à l'état appliqué.

3. Système d'aiguille de ponction (1) selon l'une quelconque des revendications précédentes, dans lequel l'optique (16) est appliquée dans la deuxième constellation au niveau du raccordement axial (32) de l'adaptateur à plusieurs voies (30).

4. Système d'aiguille de ponction (1) selon l'une quelconque des revendications précédentes, dans lequel le tuyau de protection (40) fait saillie depuis la tige (2) dans la deuxième constellation.

5. Système d'aiguille de ponction (1) selon la revendication 4, avec une fibre laser (44) pour une introduction dans le tuyau de protection (42) qui présente un couplage qui peut être relié à un couplage correspondant de la fibre laser (44).

6. Système d'aiguille de ponction (1) selon l'une quelconque des revendications précédentes, avec une autre tige (50) avec un diamètre plus grand que celui de ladite première tige (2) et un dilatateur (48).

7. Système d'aiguille de ponction (1) selon l'une quelconque des revendications précédentes, avec un désintégrateur mécanique.

8. Système d'aiguille de ponction (1) selon l'une quelconque des revendications précédentes, dans lequel un diamètre externe de la tige (2) est inférieur à 2,0 mm, préférentiellement inférieur à 1,7 mm, le plus préférentiellement d'environ 1,65 mm.
